# EUROPÄISCHE PATENTSCHRIFT

(11) **EP 1 535 021 B1**
(45) Veröffentlichungstag und Bekanntmachung des Hinweises auf die Patenterteilung: **07.06.2006**
(21) Anmeldenummer: 03753354.4
(22) Anmeldetag: 08.08.2003
(51) Int. Cl.: G01B 7/28

(54) **VORRICHTUNG ZUR ERFASSUNG DER KONTUR EINER OBERFLÄCHE EINER KNÖCHERNEN STRUKTUR**
DEVICE FOR RECORDING THE SHAPE OF A SURFACE OF A BONY STRUCTURE
DISPOSITIF POUR DETERMINER LE CONTOUR D'UNE SURFACE DE STRUCTURE OSSEUSE

(30) Priorität: 05.09.2002 DE 10241069
(43) Veröffentlichungstag der Anmeldung: 01.06.2005
(73) Patentinhaber: Aesculap AG & Co. KG, 78532 Tuttlingen (DE)
(72) Erfinder: GÖGGELMANN, Andreas, 74379 Ingersheim (DE); KOZAK, Josef, 78532 Tuttlingen (DE)
(74) Vertreter: Boehme, Ulrich
(86) Internationale Anmeldenummer: PCT/EP2003/008856
(87) Internationale Veröffentlichungsnummer: WO 2004/023068

(56) Entgegenhaltungen:
- EP-A- 0 944 237
- DE-A- 10 105 822
- DE-C- 19 851 153
- US-A- 5 198 877
- US-B1- 6 236 875
- US-B1- 6 409 686

## Beschreibung

Die Erfindung betrifft eine Vorrichtung zur Erfassung der Kontur einer Oberfläche einer knöchernen Struktur.

Die Kenntnis der Kontur von unregelmäßigen Oberflächen einer knöchernen Struktur, insbesondere der Kontur von Vertiefungen, ist in vielen Fällen Voraussetzung dafür, daß diese Oberfläche bearbeitet werden kann. Beispielsweise ist es notwendig, bei der Implantation von Endoprothesen den genauen Verlauf der Oberfläche der knöchernen Struktur zu kennen, um dementsprechend eine Anpassung von Implantaten, eine notwendige Nachbearbeitung der knöchernen Struktur oder eine geeignete Auswahl unter verschiedenen zur Verfügung stehenden Implantaten zu treffen. In der US 6,409,686 B1 ist eine Vorrichtung mit einem mechanisch bewegbaren Arm beschrieben, die zur Abtastung der Oberfläche eines Körpers dient. Die dabei eingestellte Position des Armes und eines daran gelagerten Schiebers müssen abgelesen und in ein Rechnersystem übertragen werden. Dies ist kompliziert und kann außerdem zu Fehleingaben führen.

Für den Straßenbau ist ein Fahrbahnvermessungssystem bekannt, bei dem die Oberfläche der Fahrbahn über Abstandsdetektoren ermittelt wird, wobei die Lage der Abstandsdetektoren relativ zu einer stationären Meßeinrichtung laufend bestimmt wird (DE 198 51 153 C1). Dieses System ist jedoch nicht dazu bestimmt, die Oberfläche einer knöchernen Struktur zu erfassen, insbesondere würden sich Fehlmessungen ergeben, wenn die auszumessende Oberfläche ihre Position im Raum verändern sollte.

Der Erfindung liegt die Aufgabe zugrunde, eine Vorrichtung der gattungsgemäßen Art zur Verfügung zu stellen, mit der auch bei unregelmäßigen Konturen der Oberfläche diese Kontur einfach und zuverlässig bestimmt werden kann.

Diese Aufgabe wird bei einer Vorrichtung der eingangs beschriebenen Art erfindungsgemäß dadurch gekennzeichnet, daß die Vorrichtung umfaßt:
- einen Träger, an dem mindestens ein den Abstand zwischen dem Träger und einem Ort der Oberfläche der knöchernen Struktur bestimmendes Abstandsmeßelement angeordnet ist,
- ein Navigationssystem mit am Träger und an der knöchernen Struktur festzulegenden Markierelementen, deren Position durch das Navigationssystem bestimmbar ist, und
- eine Datenverarbeitungseinrichtung, die aus den Positionsdaten der knöchernen Struktur und des Trägers und den Abstandsmessungen des oder der Abstandsmeßelemente die Kontur der Oberfläche berechnet.

Es wird also ein Träger verwendet, der relativ zu der knöchernen Struktur und zu der bestimmten Oberfläche eine bestimmbare Position einnimmt, und an dem Träger wird durch mindestens ein Abstandselement der Abstand der Oberfläche vom Träger bestimmt. Die Datenverarbeitungseinrichtung kann aus der Relativposition des Trägers zu der knöchernen Struktur einerseits und dem Abstand der Oberfläche zum Träger andererseits die Kontur der Oberfläche bestimmen, wenn eine derartige Messung an unterschiedlichen Stellen der Oberfläche durchgeführt wird.

Im einfachsten Fall läßt sich dies erreichen mit einem Träger mit einem einzigen Abstandsmeßelement, welches am Träger fest angeordnet ist, in diesem Fall wird der Träger mit dem Abstandsmeßelement über die zu bestimmende Kontur geführt, die Kontur wird auf diese Weise punktweise ausgemessen und von der Datenverarbeitungseinrichtung bestimmt.

Natürlich ist dieses Verfahren relativ umständlich, und die Genauigkeit hängt auch davon ab, wie sorgfältig der Benutzer den Träger über die gesamte zu erfassende Oberfläche bewegt.

Eine wesentliche Verbesserung ergibt sich, wenn gemäß einer bevorzugten Ausführungsform am Träger mehrere Abstandsmeßelemente über eine Fläche verteilt fest angeordnet sind, deren Anordnung am Träger in der Datenverarbeitungseinrichtung gespeichert ist oder dieser laufend übermittelt wird. Auf diese Weise können die am Träger angeordneten Abstandsmeßelemente gleichzeitig an verschiedenen Punkten der Oberfläche deren Abstand vom Träger bestimmen, und aus der Relativpositionierung der Abstandsmeßelemente zueinander und den einzelnen Abstandsmeßwerten kann die Datenverarbeitungseinrichtung dann die Kontur der Oberfläche bestimmen. Wenn die Zahl der Abstandsmeßelemente genügend groß ist und diese die Kontur der zu messenden Oberfläche genügend vollständig überdecken, kann ohne Bewegung des Trägers die Kontur berechnet werden, es genügt also, den Träger einmal gegenüber der Oberfläche zu positionieren und dann eine Messung durchzuführen, die sofort eine Konturbestimmung der gesamten Oberfläche ermöglicht.

Es ist aber auch möglich, den Träger, der mehrere Abstandsmeßelemente trägt, zu nacheinander folgenden Abstandsmessungen relativ zur Oberfläche zu bewegen, so daß die Abstandsmeßelemente bei jeder Messung einem anderen Gebiet der Oberfläche gegenüberstehen und dort eine Abstandsmessung durchführen. Da die Position des Trägers relativ zum Körper durch das Navigationssystem jederzeit bestimmt ist, kann auch bei jeder Position des Trägers eine Zuordnung der Abstandsmessungen zu einer bestimmten Stelle der Oberfläche erfolgen, so daß durch in dieser Weise aufeinanderfolgende Messungen die Zahl der Oberflächenmeßpunkte erhöht werden kann. Auf diese Weise kann mit einer unter Umständen relativ geringen Anzahl von Abstandsmeßelementen durch mehrere aufeinanderfolgende Messungen trotzdem eine Bestimmung der Kontur der Oberfläche mit großer Genauigkeit erfolgen.

Bei einer weiteren Anordnung ist es auch möglich, daß das Abstandsmeßelement am Träger in verschiedene Positionen verfahrbar ist und daß der jeweiligen Position des Abstandsmeßelements am Träger entsprechende Daten der Datenverarbeitungsanlage laufend übermittelt werden.

Bei dieser Ausgestaltung wird also die Oberfläche von dem beweglichen Abstandsmeßelement abgefahren oder gescannt. Dabei können an einem solchen Träger auch mehrere derartige verfahrbare Abstandsmeßelemente vorgesehen sein, die gleichzeitig oder nacheinander am Träger verschoben werden. Die Verschiebung kann eine körperliche Verschiebung sein, grundsätzlich wäre es auch möglich, in einem größeren Raster von Abstandsmeßelementen mit diesen nacheinander Messungen durchzuführen, so daß die aktiven Meßpunkte längs des Trägers bewegt werden und nicht die Abstandsmeßelemente selbst.

In allen Fällen ist von Bedeutung, daß der Benutzer den Träger gegenüber der zu bestimmenden Oberfläche nicht in eine definierte Position bringen muß, es genügt, wenn die Position des Trägers so gewählt wird, daß die Abstandsmeßelemente den Abstand zur Oberfläche bestimmen können, und auch Bewegungen des Trägers während der Abstandsmessungen sind unschädlich, da diese Bewegungen vom Navigationssystem erfaßt und von der Datenverarbeitungsanlage bei der Berechnung der Kontur der Oberfläche berücksichtigt werden können.

Gemäß einer bevorzugten Ausführungsform ist vorgesehen, daß das Abstandsmeßelement einen beweglichen, an der Oberfläche anlegbaren Tastkörper aufweist, dessen unterschiedliche Stellungen durch einen Sensor erfaßt und der Datenverarbeitungsanlage übermittelt werden. Bei dieser Art der Abstandsbestimmung wird also eine berührende Messung vorgenommen.

Der Tastkörper kann dabei in unterschiedlichster Weise ausgestaltet werden, beispielsweise kann der Tastkörper ein längsverschieblicher Stift sein, der so weit ausgefahren wird, bis er an seinem freien Ende an der Oberfläche anliegt. Denkbar wären auch unterschiedlich weit in eine Führung am Träger einschiebbare Kugelkörper, verschwenkbare Tastarme etc.

Der Sensor zur Bestimmung der Position des Tastkörpers kann ebenfalls in sehr unterschiedlicher Weise ausgestaltet sein, es kann sich beispielsweise um einen elektrisch arbeitenden Sensor handeln, insbesondere eine elektrische Widerstandsmeßeinrichtung, eine elektrische Magnetfeldmeßeinrichtung, eine elektrische Induktivitäts- oder Kapazitätsmeßeinrichtung, oder eine andere elektrische Meßeinrichtung, die die Lage eines Körpers relativ zu einem anderen bestimmen kann.

Es ist auch möglich, mechanische Meßeinrichtungen zu verwenden, beispielsweise eine Druck- oder Dehnungsmeßeinrichtung. In anderen Fällen kann eine optische Meßeinrichtung verwendet werden, hier sind insgesamt dem Fachmann eine Vielzahl von Möglichkeiten an die Hand gegeben, wie er die Positionsveränderung des Tastkörpers relativ zum Träger feststellen kann.

Bei einer anderen bevorzugten Ausführungsform wird der Abstand berührungslos bestimmt. Insbesondere kann dabei vorgesehen sein, daß das Abstandsmeßelement eine Strahlung aussendet und die von der Oberfläche reflektierte Strahlung empfängt und daß aus der Laufzeit der Strahlung zwischen Aussenden und Empfang der Abstand zwischen Abstandsmeßelement und Oberfläche bestimmt wird. Diese Strahlung kann beispielsweise eine Ultraschallstrahlung sein oder eine elektromagnetische Strahlung, insbesondere eine Infrarotstrahlung oder eine Laserstrahlung.

Besonders vorteilhaft ist es, wenn der Träger die Form einer Platte hat.

Die nachfolgende Beschreibung bevorzugter Ausführungsformen der Erfindung dient im Zusammenhang mit der Zeichnung der näheren Erläuterung. Es zeigen:
- Figur 1:: eine Draufsicht auf eine Konturerfassungsvorrichtung mit einer Vielzahl von auf konzentrischen Kreisen angeordneten Abstandsmeßelementen;
- Figur 2:: eine Schnittansicht längs Linie 2-2 in Figur 1;
- Figur 3:: eine Schnittansicht durch ein erstes bevorzugtes Ausführungsbeispiel eines Abstandsmeßelementes mit einem magnetischen Induktionssensor;
- Figur 4:: eine Ansicht ähnlich Figur 3 mit einem druckempfindlichen Sensor;
- Figur 5:: eine Ansicht ähnlich Figur 3 mit einem Ultraschall-Abstandsmeßelement;
- Figur 6:: eine Ansicht ähnlich Figur 5 mit einem Laserstrahl-Abstandsmeßelement und
- Figur 7:: eine schematische Seitenansicht eines am Träger der Konturerfassungsvorrichtung verschieblich gelagerten Abstandsmeßelementes.

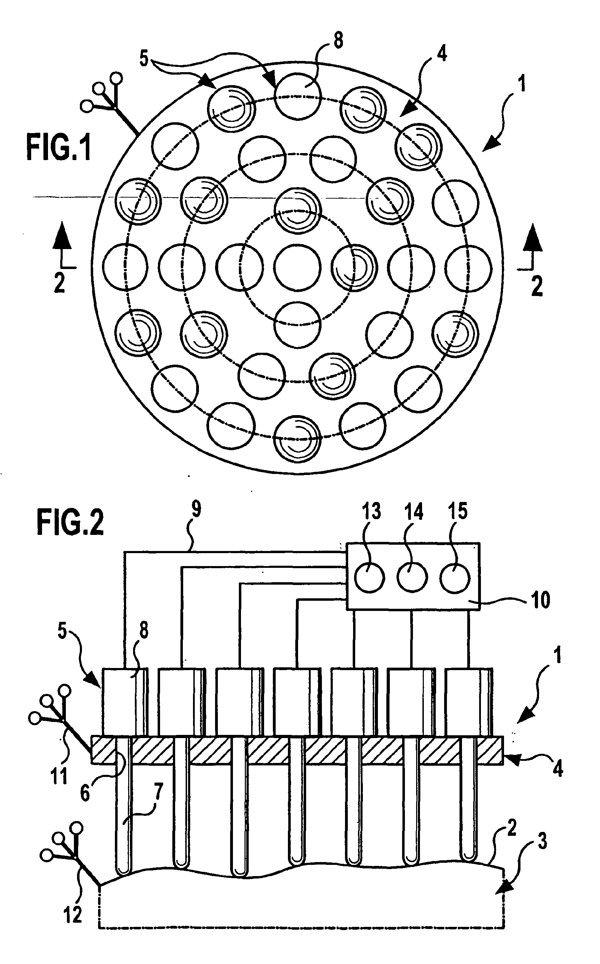

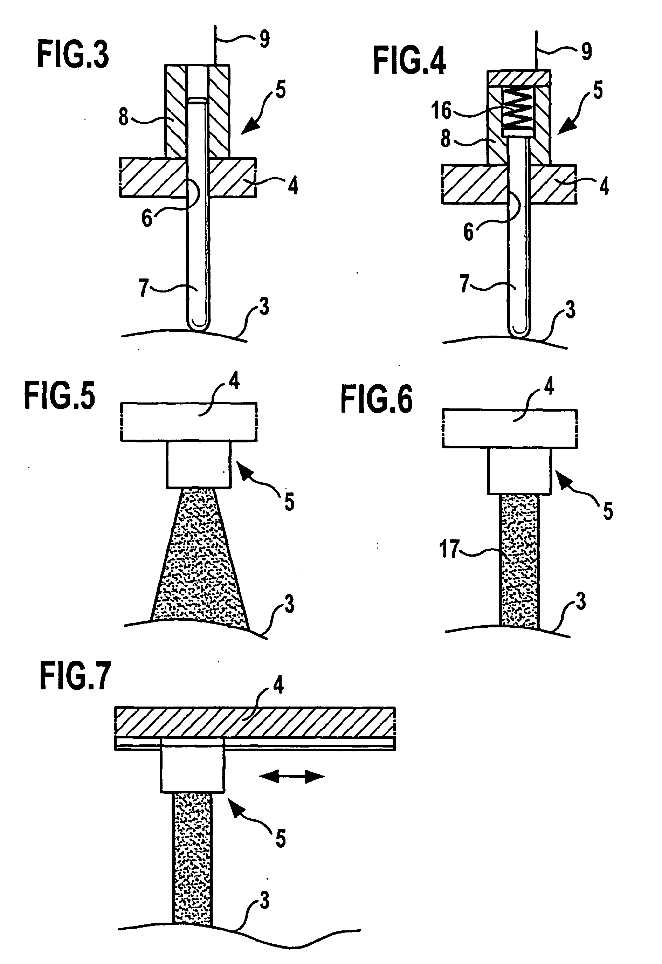

Die in den Figuren 1 und 2 dargestellte Vorrichtung 1 dient der Erfassung der Kontur einer Oberfläche 2 einer knöchernen Struktur 3. Die Vorrichtung 1 umfaßt einen plattenförmigen Träger 4, der im dargestellten Ausführungsbeispiel einen kreisförmigen Querschnitt aufweist. Auf konzentrischen Kreisen zum Mittelpunkt des Trägers 4 sind eine Vielzahl von Abstandsmeßelementen 5 angeordnet, die jeweils einen durch eine Öffnung 6 des Trägers 4 hindurchragenden und senkrecht zur Ausdehnung des Trägers 4 verschieblichen Taststift 7 und einen Sensor 8 aufweisen, der auf der Oberseite des Trägers 4 fest angeordnet ist und in den der Taststift 7 mehr oder weniger tief eingeschoben werden kann.

Jeder Sensor 8 bestimmt, wie weit der Taststift 7 in ihn eingeschoben ist, er bestimmt also mit anderen Worten, wie weit der Taststift 7 nach unten aus dem Träger 4 herausragt. Ein dieser Einschubtiefe entsprechendes elektrisches Signal wird über eine jedem Sensor 8 zugeordnete Leitung 9 einer Datenverarbeitungsanlage 10 zugeführt, die auf diese Weise von allen Sensoren 8 Daten darüber erhält, wie weit der Taststift 7 des entsprechenden Abstandsmeßelementes 5 aus dem Träger 4 nach unten ausgefahren ist.

An dem Träger 4 und an der knöchernen Struktur 3 ist jeweils ein Markierelement 11 bzw. 12 festgelegt, dieses kann in an sich bekannter Weise drei kugelförmige Reflexionskörper für Ultrarotstrahlung umfassen. Der Datenverarbeitungseinrichtung 10 ist ein Navigationssystem mit drei Sende- und Empfangseinrichtungen 13, 14, 15 zugeordnet. Diese senden beispielsweise Ultrarotstrahlung aus, die an den Markierelementen 11, 12 reflektiert wird und die dann wieder von den Sende- und Empfangseinrichtungen 13, 14, 15 empfangen wird. Aus den Laufzeitunterschieden kann dieses Navigationssystem den Abstand und die Lage der beiden Markierelemente 11, 12 relativ zum Navigationssystem bestimmen und damit die Lage und Orientierung sowohl des Trägers 4 als auch der knöchernen Struktur 3 im Raum. Diese Positionsdaten liefert das Navigationssystem an die Datenverarbeitungseinrichtung 10.

Wenn man den Träger 4 neben der Oberfläche 2 anordnet, deren Kontur zu bestimmen ist, werden die Taststifte 7 sich aufgrund der Schwerkraft so weit verschieben, bis sie jeweils an der Oberfläche 2 anliegen, entsprechend der Kontur der Oberfläche 2 werden die Taststifte 7 also unterschiedlich weit aus dem Träger 4 ausgefahren, wie dies in der Darstellung der Figur 2 erkennbar ist. Diese Position der Taststifte 7 wird von den Sensoren 8 festgestellt und an die Datenverarbeitungseinrichtung 10 weitergegeben, die dann aus diesen Daten, den Positionsdaten der Abstandsmeßelemente 5 auf dem Träger 4, die in einem Speicher der Datenverarbeitungseinrichtung 10 abgelegt sein können, und den Positionsdaten des Trägers 4 und der knöchernen Struktur 3 punktweise die Kontur der Oberfläche 2 berechnen kann. Aufgrund der Vielzahl von Abstandsmeßelementen 5 am Träger 4 erhält man auf diese Weise ein recht zuverlässiges Bild über die Kontur der Oberfläche 2. Die Genauigkeit kann einmal verbessert werden durch Verwendung einer größeren Anzahl von Abstandsmeßelementen 5, die dann dichter aneinander heranrücken, aber auch dadurch, daß man mehrere derartige Messungen nacheinander durchführt, bei denen der Träger 4 relativ zur Oberfläche 2 jeweils geringfügig in seiner Position verschoben wird. Auf diese Weise legen sich die Taststifte 7 bei jeder Messung an einer anderen Stelle der Oberfläche 2 an und fügen den vorhergehenden Messungen zusätzliche Positionsmessungen der Oberflächenstruktur hinzu.

Die Sensoren können sehr unterschiedlich ausgebildet sein, in Figur 3 ist beispielsweise ein Abstandsmeßelement 5 dargestellt, bei dem die elektrischen Eigenschaften des den Taststift 7 hülsenförmig umgebenden Sensors 8 durch die unterschiedliche Einschubtiefe des Taststiftes 7 verändert werden, beispielsweise die Induktivität einer Spule oder der magnetische Fluß in einem Ringmagneten etc.

Beim Ausführungsbeispiel der Figur 4 wird der Taststift 7 gegenüber einer Feder 16 in das Innere des Sensors 8 eingeschoben, die Dehnung oder Stauchung der Feder kann über einen Dehnmeßstreifen, über eine Druckmeßdose oder ähnliche Elemente bestimmt werden.

Beim Ausführungsbeispiel der Figur 5 ist schematisch ein berührungsloses Abstandsmeßelement 5 beschrieben, welches eine Ultraschallstrahlung abstrahlt. Der Sensor 8 empfängt die an der Oberfläche 2 reflektierte Ultraschallstrahlung und führt der Datenverarbeitungseinrichtung 10 ein Signal zu, welches die Laufzeit der Ultraschallstrahlung zwischen Aussenden und Empfang repräsentiert. Aus dieser Laufzeit läßt sich der Abstand der Oberfläche 2 vom Abstandsmeßelement 5 bestimmen.

Anstelle der Ultraschallstrahlung könnte auch eine elektromagnetische Strahlung verwendet werden, beispielsweise eine Infrarotstrahlung oder eine Laserstrahlung, in Figur 6 ist ein Abstandselement 5 schematisch dargestellt, welches einen Laserstrahl 17 aussendet.

Während bei den bisher beschriebenen Ausführungsbeispielen die Abstandsmeßelemente 5 am Träger 4 fest angeordnet sind, wäre es auch denkbar, eines oder mehrere Abstandsmeßelemente 5 am Träger 4 verschieblich zu lagern, wie dies in Figur 7 schematisch dargestellt ist. Die jeweilige Position des Abstandsmeßelementes 5 am Träger 4 wird dann durch geeignete Sensoren zusätzlich bestimmt und ebenfalls über eine entsprechende Leitung der Datenverarbeitungseinrichtung 10 zugeführt, so daß längs des Verschiebeweges in unterschiedlichen Positionen Abstandsdaten und Positionsdaten des Abstandsmeßelementes 5 am Träger 4 in der Datenverarbeitungseinrichtung 10 verarbeitet werden. Es ist damit möglich, durch das bewegliche Abstandsmeßelement 5 die Oberfläche 2 zu überfahren und nacheinander an verschiedenen Stellen dieser Oberfläche 2 Abstandsmessungen durchzuführen. Dabei können an dem Träger 4 ein derartiges verfahrbares Abstandsmeßelement 5 oder aber auch mehrere angeordnet werden, grundsätzlich wäre es auch möglich, feste Abstandsmeßelemente 5 mit in dieser Weise beschriebenen beweglichen Abstandsmeßelementen 5 an einem Träger 4 zu kombinieren.

## Patentansprüche

1. Vorrichtung zur Erfassung der Kontur einer Oberfläche (2) einer knöchernen Struktur (3), **dadurch gekennzeichnet, daß** sie umfaßt:
einen Träger (4), an dem mindestens ein den Abstand zwischen dem Träger (4) und einem Ort der Oberfläche (2) der knöchernen Struktur(3) bestimmendes Abstandsmeßelement (5) angeordnet ist,
ein Navigationssystem (11, 12; 13, 14, 15) mit am Träger (4) und an der knöchernen Struktur (3) festzulegenden Markierelementen, deren Position durch das Navigationssystem bestimmbar ist und
eine Datenverarbeitungsanlage (10), die aus den Positionsdaten der knöchernen Struktur (3) und des Trägers (4) und den Abstandsmessungen des oder der Abstandsmeßelemente (5) die Kontur der Oberfläche (2) berechnet.

2. Vorrichtung nach Anspruch 1, **dadurch gekennzeichnet, daß** am Träger (4) mehrere Abstandsmeßelemente (5) über eine Fläche verteilt fest angeordnet sind, deren Anordnung am Träger (4) in der Datenverarbeitungsanlage (10) gespeichert ist oder dieser laufend übermittelt wird.

3. Vorrichtung nach Anspruch 1, **dadurch gekennzeichnet, daß** das Abstandselement (5) am Träger (4) in verschiedene Positionen verfahrbar ist und daß der jeweiligen Position des Abstandsmeßelementes (5) am Träger (4) entsprechende Daten der Datenverarbeitungsanlage (10) laufend übermittelt werden.

4. Vorrichtung nach einem der voranstehenden Ansprüche, **dadurch gekennzeichnet, daß** das Abstandsmeßelement (5) einen beweglichen, an der Oberfläche (2) anlegbaren Tastkörper (7) aufweist, dessen unterschiedliche Stellungen durch einen Sensor (8) erfaßt und der Datenverarbeitungsanlage (10) übermittelt werden.

5. Vorrichtung nach Anspruch 4, **dadurch gekennzeichnet, daß** der Tastkörper (7) ein längsverschieblicher Stift ist.

6. Vorrichtung nach Anspruch 4 oder 5, **dadurch gekennzeichnet, daß** der Sensor (8) zur Bestimmung der Position des Tastkörpers (7) eine elektrische Widerstandsmeßeinrichtung ist.

7. Vorrichtung nach Anspruch 4 oder 5, **dadurch gekennzeichnet, daß** der Sensor (8) zur Bestimmung der Position des Tastkörpers (7) eine Magnetfeldmeßeinrichtung ist.

8. Vorrichtung nach Anspruch 4 oder 5, **dadurch gekennzeichnet, daß** der Sensor (8) zur Bestimmung der Position des Tastkörpers (7) eine elektrische Induktivitäts- oder Kapazitätsmeßeinrichtung ist.

9. Vorrichtung nach Anspruch 4 oder 5, **dadurch gekennzeichnet, daß** der Sensor (8) zur Bestimmung der Position des Tastkörpers (7) eine mechanische Druck- oder Dehnungsmeßeinrichtung ist.

10. Vorrichtung nach Anspruch 4 oder 5, **dadurch gekennzeichnet, daß** der Sensor (8) zur Bestimmung der Position des Tastkörpers (7) eine optische Meßeinrichtung ist.

11. Vorrichtung nach einem der Ansprüche 1 bis 3, **dadurch gekennzeichnet, daß** das Abstandsmeßelement (5) eine Strahlung (17) aussendet und die von der Oberfläche reflektierte Strahlung empfängt und daß aus der Laufzeit der Strahlung zwischen Aussenden und Empfang der Abstand zwischen Abstandsmeßelementen (5) und Oberflächen (2) bestimmt wird.

12. Vorrichtung nach Anspruch 11, **dadurch gekennzeichnet, daß** die Strahlung Ultraschallstrahlung ist.

13. Vorrichtung nach Anspruch 11, **dadurch gekennzeichnet, daß** die Strahlung (17) elektromagnetische Strahlung ist.

14. Vorrichtung nach einem der voranstehenden Ansprüche, **dadurch gekennzeichnet, daß** der Träger (4) die Form einer Platte hat.

## Claims

1. Apparatus for recording the contour of a surface (2) of a bone structure (3), **characterized in that** it comprises:
a carrier (4) on which at least one distance measuring element (5) determining the distance between the carrier (4) and a place on the surface (2) of the bone structure (3) is disposed,
a navigation system (11, 12; 13, 14, 15) with marking elements which are to be fixed on the carrier (4) and on the bone structure (3) and the position of which can be determined by the navigation system, and
a data-processing system (10), which calculates the contour of the surface (2) from the positional data of the bone structure (3) and of the carrier (4) and the distance measurements of the distance measuring element or elements (5).

2. Apparatus according to Claim 1, **characterized in that** a number of distance measuring elements (5) are fixedly disposed on the carrier (4), distributed over a surface area, and their disposition on the carrier (4) is stored in the data-processing system (10) or continually transmitted to the latter.

3. Apparatus according to Claim 1, **characterized in that** the distance measuring element (5) is able to move on the carrier (4) into various positions and **in that** data corresponding to the respective position of the distance measuring element (5) on the carrier (4) are continually transmitted to the data-processing system (10).

4. Apparatus according to one of the preceding claims, **characterized in that** the distance measuring element (5) has a movable feeling element (7) which can be placed on the surface (2) and the different positions of which are recorded by a sensor (8) and transmitted to the data-processing system (10).

5. Apparatus according to Claim 4, **characterized in that** the feeling element (7) is a longitudinally displaceable pin.

6. Apparatus according to Claim 4 or 5, **characterized in that** the sensor (8) for determining the position of the feeling element (7) is an electrical resistance measuring device.

7. Apparatus according to Claim 4 or 5, **characterized in that** the sensor (8) for determining the position of the feeling element (7) is a magnetic field measuring device.

8. Apparatus according to Claim 4 or 5, **characterized in that** the sensor (8) for determining the position of the feeling element (7) is an electrical inductance or capacitance measuring device.

9. Apparatus according to Claim 4 or 5, **characterized in that** the sensor (8) for determining the position of the feeling element (7) is a mechanical pressure or strain measuring device.

10. Apparatus according to Claim 4 or 5, **characterized in that** the sensor (8) for determining, the position of the feeling element (7) is an optical measuring device.

11. Apparatus according to one of Claims 1 to 3, **characterized in that** the distance measuring element (5) emits a radiation (17) and receives the radiation reflected from the surface and **in that** the distance between the distance measuring elements (5) and the surfaces (2) is determined from the transit time of the radiation between emission and reception.

12. Apparatus according to Claim 11, **characterized in that** the radiation is ultrasonic radiation.

13. Apparatus according to Claim 11, **characterized in that** the radiation (17) is electromagnetic radiation.

14. Apparatus according to one of the preceding claims, **characterized in that** the carrier (4) is in the form of a plate.

## Revendications

1. Dispositif pour détecter le contour d'une surface (2) d'une structure osseuse (3), **caractérisé en ce qu'**il comprend :
un support (4) sur lequel est agencé au moins un élément de mesure d'espacement (5) déterminant l'espacement entre le support (4) et un emplacement de la surface (2) de la structure osseuse (3),
un système de navigation (11, 12 ; 13, 14, 15) comportant des éléments de marquage à fixer sur le support (4) et sur la structure osseuse (3), dont la position peut être déterminée par le système de navigation,
une installation de traitement de données (10) qui calcule le contour de la surface (2) à partir des données de position de la structure osseuse (3) et du support (4) et à partir des mesures d'espacement du ou des éléments de mesure d'espacement (5).

2. Dispositif selon la revendication 1, **caractérisé en ce que** sur le support (4), plusieurs éléments de mesure d'espacement (5) sont agencés en étant répartis de façon fixe sur une surface, dont l'agencement sur le support* est mémorisé dans l'installation de traitement de données (10) ou est transmis en permanence à celle-ci.

3. Dispositif selon la revendication 1, **caractérisé en ce que** l'élément d'espacement (5) sur le support (4) peut être déplacé dans différentes positions et **en ce que** des données qui correspondent à la position respective de l'élément de mesure d'espacement (5) sur le support sont transmises en permanence à l'installation de traitement de données (10).

4. Dispositif selon l'une des revendications précédentes, **caractérisé en ce que** l'élément de mesure d'espacement (5) présente un corps de palpage (7) mobile applicable sur la surface (2), dont différentes positions sont détectées par un capteur (8) et transmises à l'installation de traitement de données (10).

5. Dispositif selon la revendication 4, **caractérisé en ce que** le corps de palpage (7) est une tige à déplacement longitudinal.
* (4)

6. Dispositif selon la revendication 4 ou 5, **caractérisé en ce que** le capteur (8) servant à déterminer la position du corps de palpage (7) est un dispositif de mesure de résistance électrique.

7. Dispositif selon la revendication 4 ou 5, **caractérisé en ce que** le capteur (8) servant à déterminer la position du corps de palpage (7) est un dispositif de mesure de champ magnétique.

8. Dispositif selon la revendication 4 ou 5, **caractérisé en ce que** le capteur (8) servant à déterminer la position du corps de palpage (7) est un dispositif de mesure d'induction ou de capacité.

9. Dispositif selon la revendication 4 ou 5, **caractérisé en ce que** le capteur (8) servant à déterminer la position du corps de palpage (7) est un dispositif de mesure mécanique de pression ou d'élongation.

10. Dispositif selon la revendication 4 ou 5, **caractérisé en ce que** le capteur (8) servant à déterminer la position du corps de palpage (7) est un dispositif de mesure optique.

11. Dispositif selon l'une des revendications 1 à 3, **caractérisé en ce que** l'élément de mesure d'espacement (5) émet un rayonnement (17) et reçoit le rayonnement réfléchi par la surface, et **en ce que** l'espacement entre l'élément de mesure d'espacement (5) et les surfaces (2) est mesuré à partir de la durée de propagation du rayonnement entre l'émission et la réception.

12. Dispositif selon la revendication 11, **caractérisé en ce que** le rayonnement est du rayonnement ultrasonore.

13. Dispositif selon la revendication 11, **caractérisé en ce que** le rayonnement (17) est du rayonnement électromagnétique.

14. Dispositif selon l'une des revendications précédentes, **caractérisé en ce que** le support (4) a la forme d'une plaque.
